(19) Europäisches Patentamt
European Patent Office
Office européen des brevets

(11) **EP 4 273 543 B1**

(12) **EUROPEAN PATENT SPECIFICATION**

(45) Date of publication and mention
of the grant of the patent:
**15.01.2025 Bulletin 2025/03**

(21) Application number: **22382436.8**

(22) Date of filing: **06.05.2022**

(51) International Patent Classification (IPC):
$G01N\ 33/00$ (2006.01)    $A01G\ 7/00$ (2006.01)
$G01N\ 27/04$ (2006.01)

(52) Cooperative Patent Classification (CPC):
**G01N 33/0098; A01G 7/00;** G01N 27/04

(54) **SYSTEM AND METHOD FOR THE EVALUATION OF THE HYDRIC STATE OF A PLANT**

SYSTEM UND VERFAHREN ZUR BEWERTUNG DES WASSERZUSTANDES EINER PFLANZE

SYSTÈME ET PROCÉDÉ POUR L'ÉVALUATION DE L'ÉTAT HYDRIQUE D'UNE INSTALLATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**08.11.2023 Bulletin 2023/45**

(73) Proprietor: **HYDROSTAT CEN, SL
07320 Santa Maria, Illes Balears (ES)**

(72) Inventors:
• **BOSCH ROS, David**
**08207 Sabadell (ES)**
• **SANZ GÓMEZ, Álvaro**
**07007 Palma de Mallorca. (ES)**
• **TORTOSA MONTOJO, Ignacio**
**07013 Palma de Mallorca (ES)**
• **PALERM LLABRÉS, Antoni**
**07550 Son Servera (ES)**
• **MARTÍ SAURAS, José Mª**
**07320 Binissalem (ES)**
• **ESCALONA LORENZO, José Mariano**
**07008 Palma de Mallorca (ES)**
• **MEDRANO GIL, Hipólito**
**07014 Palma de Mallorca (ES)**

(74) Representative: **Torner, Juncosa I Associats, SL
C / Pau Claris, 108, 1r 1a
08009 Barcelona (ES)**

(56) References cited:
EP-A1- 3 104 135        WO-A1-2007/039537
WO-A1-2022/039007       CN-A- 114 020 065
DE-A1- 102006 026 557

**Description**

Technical Field

The present invention concerns a system, sometimes implemented as a device, and a method, for the evaluation of the hydric state of a plant, in the stem, branch or trunk.

Background of the Invention

US9857391 B2 discloses a plant water dynamics sensor comprising a heater-equipped temperature probe including a temperature sensor and a heater; a temperature probe including a temperature sensor; an electrical resistance probe including an electrical resistance measurement electrode; and a support that supports the probes while the probes are aligned parallel to each other. The position of a xylem XY can be detected based on an electrical resistance measured at the electrical resistance probe, so that each of the temperature sensors can be arranged correctly in a position at a phloem PH or at the xylem XY. This facilitates attachment of a plant water dynamics sensor and water dynamics in a plant can be measured with high accuracy.

JPH06273434 discloses a measuring apparatus of the flow velocity of water content mainly inside the trachea of a plant. The measuring apparatus is composed of a measurement part in which a heater protruding from a mounting seat is installed, in which a first temperature sensor and a second temperature sensor are installed so as to protrude from the mounting seat toward the same direction as the heater and so as to sandwich the heater, and in which a third temperature sensor is installed between the first temperature sensor and the heater. The measuring apparatus is composed of a computer with a built-in program which measures the temperature difference between the first temperature sensor and the second temperature sensor and the temperature difference between the first temperature sensor and the third temperature sensor by the measurement part and which computes a flow velocity by a computation formula stored in advance by transmitting the product of both temperature differences.

KR101996404B1 relates to a portable type and installation type tree management device capable of managing trees by species scientifically and systematically. The device comprises a first measuring unit including a pair of electrodes each having a plurality of needles for insertion into a tree trunk, and measuring electrical conductivity; a diagnosis unit diagnosing the health state of a tree based on the measured electrical conductivity; and a communication unit transmitting the diagnosed health state of the tree to the user.

CN111189885A relates to a trunk moisture content measuring method based on equivalent power supply internal resistance. The method comprises selecting two points on a trunk along the axial direction and respectively arranging a metal electrode a and a metal electrode b; calculating the internal resistance of a power supply formed by the metal electrode a, the metal electrode b and the trunk; and calculating the trunk moisture content through a relation model MC=f(Ri) between the trunk moisture content and the power supply internal resistance. CN113418963A discloses a trunk freeze-thaw impedance image real-time detection method and system. The method comprises the steps: constructing a multi-frequency sweep frequency method based on the impedance characteristics of a trunk, manufacturing a measurement probe comprising a plurality of conical stainless steel electrodes, and building a two-end hybrid drive excitation method based on the measurement probe, and used for acquiring trunk cross section freeze-thaw impedance characteristic information data; performing regularization processing and two-dimensional image reconstruction and correction to obtain a trunk freeze-thaw impedance two-dimensional image; performing correction to obtain a trunk freeze-thaw impedance diagram; and establishing a trunk freeze-thaw information real-time sensing model based on the freeze-thaw impedance image, and calculating the real-time freeze-thaw degree and freeze-thaw depth of the trunk in the wintering period. The system comprises a trunk cross section freeze-thaw impedance acquisition unit, a trunk freeze-thaw impedance two-dimensional image reconstruction unit, a temperature compensation unit and a trunk freeze-thaw information real-time sensing unit.

WO2022039007 discloses a known plant water content sensor (1) having a water content probe (20) with a read electrode pair (21). The water content probe comprises a water-sensitive film (23) bridging the pair of electrodes and a support (10) that supports the water content probe. The water content probe is inserted into the plant and the water content of the plant can be measured by reading the impedance or capacitance from the read electrode pair.

Despite the known solutions for the evaluation of the hydric state of a plant, i.e. the amount of water the plant has, there is still a need for alternative, faster, more reliable and accurate systems and methods.

Description of the Invention

To that end the present invention proposes, according to a first aspect, a system for evaluating the hydric state of a plant. The system comprises at least two electrodes spaced a certain distance apart, and adapted for its insertion, at a given depth, into the trunk of a plant to establish galvanic contact with the plant; at least one temperature sensor of the electrodes; an electronic unit, operatively connected to the two electrodes and the temperature sensor of the electrodes; and a processing unit, having a memory and one or more processors.

According to the present invention, the electronic unit includes a voltage measurement module which is responsible for: applying, during a determined first time interval, an electric voltage, with a determined polarity, on the electrodes; inverting, in a sequential manner, during a determined second time interval, said determined polarity; and measuring voltage values resulting from said application of electric voltage in both polarities carried out during said first and second time intervals. The temperature sensor of the electrodes, at the same time as said measurement, measures the temperature of the electrodes. Finally, the processing unit, by means of one of its processors, calculates the electrical conductance value from the applied electrical voltage and the resulting measured voltage values, and infers a value relative to the hydric state of the plant by processing the calculated conductance value and the measured electrode temperature.

Embodiments of the present invention also provide, according to a second aspect, a method for assessing the hydric state of a plant. The method comprises: inserting, at a given depth, at least two electrodes in the trunk of a plant, wherein the two electrodes maintain galvanic contact with the plant and are separated a certain distance from each other; inserting a temperature sensor of the electrodes; applying, by an electronic unit, during a first time interval and with a given polarity, an electric voltage on the electrodes; reversing, by the electronic unit, sequentially, during a second time interval, said given polarity; to measure, by the electronic unit using, simultaneously, a voltage measurement module and said electrode temperature sensor, voltage values resulting from the application of electric voltage in both polarities carried out during said first and second time intervals, and the temperature of the electrodes; calculating, by a processing unit, an electrical conductance value from the applied electrical voltage and the resulting measured voltage values; and inferring, by the processing unit, a value relating to the hydric state of the plant by processing the calculated conductance value and the measured electrode temperature.

In some embodiments, the voltage measurement module comprises a Wheatstone bridge that allows the passage of electric current resulting from the electric voltage applied across it.

In some embodiments, the system/device further comprises an analog-to-digital converter for, prior to processing, converting said resulting voltage values and the measured electrode temperature into equivalent digital signals.

In some embodiments, the electronic unit further comprises an electrical power supply (e.g., one or more batteries, photovoltaic panels, or thermoelectric generators).

In some embodiments, an ambient temperature sensor is also included. Likewise, the system/device can also include an ambient humidity sensor, a light radiation sensor, a geopositioning system, an internal operational error checking system and/or a real-time clock.

In some embodiments, a system for attaching the electronic unit to the trunk of the plant is also included. Further, the electronic unit can contain a visualization interface, e.g., a screen, or alternatively or complementarily, a voice interface.

In an embodiment, the processing unit is remote to the electronic unit. In this case, both elements include a communications module, wireless or wired, and the electronic unit can also include a local processing unit. Alternatively, the processing unit is included in (i.e., local to) the electronic unit. In this case, the electronic unit comprises a communications module, wireless or wired.

In some embodiments, simultaneous to the measurement step, a measurement of an ambient temperature near the plant is also performed. Likewise, in some examples, a measurement of other parameters, such as ambient humidity, light radiation, geolocation and/or a time stamp, can also be performed.

In some embodiments, the first time interval and the second time interval have a fixed duration. In other embodiments, the application and inversion are performed until a stabilization of the electrical voltage in an area where the two electrodes are inserted.

Brief Description of the Drawings

The previous and other advantages and features will be more fully understood from the following detailed description of embodiments, with reference to the attached figures, which must be considered in an illustrative and non-limiting manner, in which:

Fig. 1 schematically illustrates an embodiment of the proposed system for measuring the hydric state of a plant.

Fig. 2 is a flow diagram illustrating a method for measuring the hydric state of a plant, according to an embodiment of the present invention.

Detailed Description of the Invention and of some Embodiments

The present invention provides a system (sometimes implemented as a device or apparatus) and a method for the evaluation of the hydric state of a plant.

Said hydric state can be objectively defined from physiological variables measured in the plant. The most widely used in the field of plant physiology and agronomy is the hydric potential. This is defined as the capacity of water molecules to move freely through a given medium. In the case of plants, it refers to the capacity to transport or mobilize water through the cellular tissue of the plant, a function and condition necessary to stay alive.

In the case of the proposal presented here, hydric potential refers to the plant's branch hydric potential. The measurement of hydric potential is carried out by applying pressure to extract water from the plant through the leaf, specifically through the petiole of a leaf. For this purpose, use is generally made of a pressure pump or Scholander chamber.

More particularly, in the present case, methodologically, the measurement is made to be representative of the branch hydric potential, causing the closure of the stomata of the leaf to be measured so that its hydric potential is equalized with that of the branch on which it is located.

Therefore, the proposal presented is related to a system and a method for the measurement of the hydric potential as a representative indicator of the hydric state of a plant.

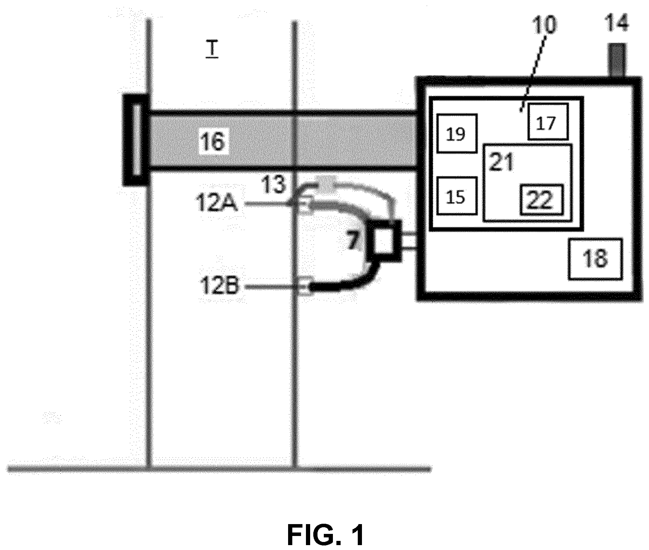

With reference now to Fig. 1, therein an embodiment of the proposed measuring system/device is illustrated. According to this particular example, the measuring system comprises an electronic unit (10), two electrodes (12A) and (12B), a temperature sensor of the electrodes (13), an ambient temperature sensor (14), and an electrical power supply (18). The measuring system is attached to the trunk (T) of the plant by means of a fastening system (16), e.g., straps, or the like.

As shown in the figure, the two electrodes (12A, 12B) are spaced a certain distance apart and connected to the temperature sensor (13) by means of a connection element (7).

The electronic unit (10), according to this particular example, includes a voltage measurement module (21), a processing unit (17), a communications unit (19), preferably wireless, and an analog/digital converter (15) for digitalization of the data/parameters obtained with the rest of the elements/sensors. The voltage module (21), as also illustrated in Fig. 1, comprises a Wheatstone bridge (22). Not limiting, since in other alternative embodiments other alternative electronic resistance measurement circuits can be used.

In addition to the aforementioned processing unit (17), a second processing unit external or remote to the measuring system/device can be included. In this case, the processing unit (17) local to the electronic unit (10) would not perform any data processing, but would only communicate the measured data/parameters to the external or remote processing unit for processing.

Although not illustrated in Fig. 1, for simplicity thereof, the proposed measurement system can include additional elements/sensors, such as, an environmental humidity sensor, a light radiation sensor, a geopositioning system, an internal operating error checking system, a real-time clock, etc.

In other embodiments, in this case not illustrated, the measurement system may not contain the ambient temperature sensor (14) or the analog/digital converter (15). Also, the electronic unit (10) may not include any processing unit with all calculations/processing being performed remotely to the measurement device.

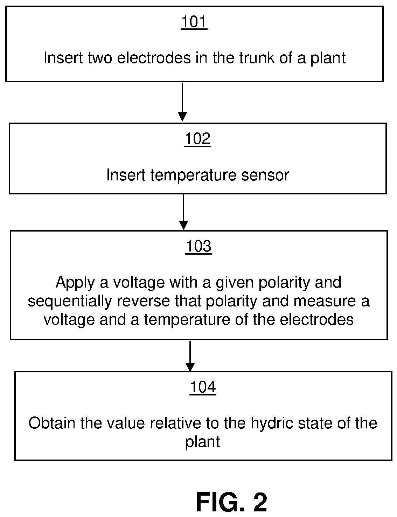

With reference now to Fig. 2, therein an embodiment of the proposed method is shown. According to this embodiment, at step 101, the two electrodes (12A, 12B) are inserted at a given depth into the trunk (T) of a plant to maintain galvanic contact with the plant. At step 102, the electrode temperature sensor (13) is inserted. In some examples, the electrode temperature sensor (13) is integrated within one of the electrodes (12A, 12B). Therefore, in these cases, steps 101 and 102 are performed at the same time.

Next, at step 103, the electronic unit (10), applies, during a first time interval, an electrical voltage at a given polarity on the electrodes (12A, 12B), inverts, sequentially, during a second time interval, said polarity, and measures, simultaneously, the temperature of the electrodes (and thus of the trunk (T) as the same are inserted therein) and some voltage values resulting from said application of electrical voltage at both polarities.

In some embodiments, the above step is performed until the electrical voltage in the area where the two electrodes (12A, 12B) are inserted is stable. For example, with a direct current injection, with a fixed applied voltage of, for example, 2.5 volts, during a time interval of 150 seconds, the measured and recorded value of voltage having passed through the electrodes and the plant, varies throughout the duration of the injection, going from values far from the voltage applied with the injection to values closer to this one; that is, they tend to resemble more the voltage at origin as the injection is extended in time. This fact responds to the phenomenon of polarization of electrically charged particles in the electric field formed between the two electrodes, with charged particles accumulating progressively. At the beginning the polarization is more accelerated and, progressively, it slows down. This slowdown in polarization and, therefore, in the variation of the measured voltage, is what is defined as signal stabilization. In other words, following the previous example, a variation of less than 0.1% of the voltage in 1% of the time, i.e. 0.0025 V in 1.5 s, being arbitrary in its conception, could be understood in the context of a stabilized signal.

In any case, and taking into account that the same degree of stabilization of the signal can occur at different times during the current injection, varying in the time in which it occurs, because the phenomenon of polarization depends, among other factors (e.g., electromagnetic radiation on the signal, temperature variation, etc.), on a greater or lesser presence of electric charges with free mobility in a fluid and, therefore, on the amount of fluid present in the area of the electric field (i.e., on the hydric state of the plant), alternatively, in other embodiments, it is preferable to set a fixed time, for instance, of approximately 150 seconds, to perform the voltage injection and measurement and to use a specific voltage data, for instance, at 149 seconds, to determine the conductance value to be used in the process of estimating the hydric state.

Following the explanation of Fig. 2, finally, at step 104, a processing unit determines the hydric state of the plant by implementing one or more algorithms on the measured data/parameters. Particularly, said one or more algorithms calculate the electrical conductance value, and with this calculated conductance value and the temperature of the electrodes the hydric state of the plant is finally inferred.

In some embodiments, and particularly if the electronic unit (10) includes said analog/digital converter (15), the measured voltage and temperature data/parameters are digitized prior to processing.

In some embodiments, at step 104, simultaneously with the other measurements, the temperature of the volume of air in contact with the temperature sensor (14), and/or the relative humidity of the volume of air in contact with the ambient humidity sensor, and/or the incident light radiation at the light radiation sensor can also be measured.

In a particular embodiment, from the resulting voltage values, a representative voltage value is selected, and from this value, the electrical conductance is calculated using the Wheatstone bridge equation:

$$\text{Conductancia} = \frac{V_{in} - V_m(1 + \frac{R_b}{R})}{V_{in} \cdot R_b + V_m(R_b + R)} \Big|$$

where: $V_{in}$ is the applied electrical voltage; $V_m$ is the measured voltage; and $R_b$ and $R$ are fixed and known resistances. Then the regression line is calculated, for example, for each day, between the series of electrical conductance and temperature measured in the trunk, for example, between the time period between 6h and 14h. Applying the formula of the regression line, the value of electrical conductance at the same temperature is obtained for each day. To obtain a value relative to the hydric state of a plant, there are two ways to proceed, which are not mutually exclusive.

INDIVIDUAL MODEL: In this case, a period of time is established in which the measurement system is installed in the plant and in which the branch water potential is also measured regularly. From a series of measurements of branch hydric potential and conductance at the same temperature, which show a linear relationship, the regression line between both parameters is obtained. Finally, the line is used to extrapolate the branch hydric potential corresponding to the conductance measurement at the same temperature measured with the measuring system, allowing to obtain, on a daily basis, a calculated value of the branch hydric potential.

GENERAL MODEL: In this case, a single regression line is constructed for a species relating the conductance at the same temperature measured with the measurement system and the branch hydric potential. Given a set of plants in which the conductance at the same temperature and the branch water potential have been measured, a plant is selected whose conductance at the same temperature - branch hydric potential relationship is particularly good (base regression). For the rest of the plants, optimization is used to obtain the value (specific for each plant/equipment) that must be added or subtracted to all the points of the regression of that plant so that it fits better to the base regression. In this way, a single regression line is obtained between the branch hydric potential and the conductance at the same temperature of the whole set of plants (general line).

This general regression can be used in new installations of measurement systems on plants of the same species. For this purpose, a single measurement of the branch hydric potential is made at the time of installation of the equipment. From this measurement and the general line, the conductance value at the same temperature is obtained, which must be added or subtracted so that the measured value of the branch hydric potential is on the general line. This value (specific to each plant/equipment) will be used in the successive conductance measurements at the same temperature to calculate, with the desired periodicity, its branch hydric potential.

The scope of present invention is defined in the claims that follow.

## Claims

1. A system for evaluating the hydric state of a plant, comprising:

   - at least two electrodes (12A, 12B) separated a certain distance from each other, and adapted for its insertion, to a given depth, into the trunk of a plant to establish a galvanic contact with the plant;
   - at least one temperature sensor of the electrodes (13);
   - an electronic unit (10), operatively connected to the two electrodes (12A, 12B) and to the temperature sensor of the electrodes (13); and
   - a processing unit including a memory and at least one processor,

   the system being **characterized in that:**

   - the electronic unit (10) includes a voltage measurement module (21) configured to:

     - apply, during a first time interval, an electric voltage, with a given polarity, on the at least two electrodes (12A, 12B);
     - invert, sequentially, during a second time interval, said given polarity; and
     - measure voltage values resulting from said application of electric voltage in both polarities carried out during said first and second time intervals;

     wherein the temperature sensor of the electrodes (13) is configured to, simultaneously with said measurement, measure a temperature of the electrodes (12A, 12B); and

wherein the at least one processor is configured to:

calculate an electrical conductance value from the applied electrical voltage and the resulting measured voltage values, and infer a value relative to the hydric state of the plant by processing the calculated conductance value and the measured electrode temperature.

2. The system of claim 1, wherein the voltage measurement module (21) comprises a Wheatstone bridge (22) adapted to allow the passage of electric current resulting from the electric voltage applied across it.

3. The system of claim 1 or 2, further comprising an analog-to-digital converter (15) adapted to, prior to processing, convert said resulting voltage values and the measured electrode temperature into equivalent digital signals.

4. The system of any one of the previous claims, wherein the electronic unit (10) further comprises an electrical power supply (18) including one or more batteries, one or more photovoltaic panels, or one or more thermoelectric generators.

5. The system of any one of the previous claims, further comprising an ambient temperature sensor (14).

6. The system of any one of the previous claims, further comprising one or more of the following elements: an ambient humidity sensor, a light radiation sensor, a geopositioning system, an internal operational error checking system and/or a real-time clock.

7. The system of any one of the previous claims, wherein the processing unit is remote to the electronic unit (10), wherein the processing unit and the electronic unit (10) each include a communications module.

8. The system of any one of the previous claims 1 to 6, wherein the processing unit is included in the electronic unit (10), wherein the electronic unit (10) further includes a communications module.

9. The system of any one of the previous claims 1 to 6, wherein the processing unit is remote to the electronic unit (10) and the electronic unit (10) includes a local processing unit (17), both processing units connected by means of a communications unit (19) of the electronic unit (10).

10. The system of any one of the previous claims, further comprising a fastening system (16) of the electronic unit (10) to the trunk of the plant.

11. The system of any one of the previous claims, wherein the electronic unit (10) includes a visualization interface.

12. A method for evaluating the hydric state of a plant, comprising:

a) inserting, at a given depth, at least two electrodes (12A, 12B) into the trunk of a plant, the two electrodes (12A, 12B) maintain a galvanic contact with the plant and are separated a certain distance from each other;
b) inserting at least one temperature sensor of the electrodes (13);
c) applying, by an electronic unit (10), during a first time interval, an electric voltage, with a given polarity, on the at least two electrodes (12A, 12B);
d) inverting, by the electronic unit (10), sequentially, during a second time interval, said given polarity;
e) measuring, by the electronic unit (10) using a voltage measurement module (21) and the temperature sensor of the electrodes (13), simultaneously, voltage values resulting from said application of electric voltage in both polarities carried out during said first and second time intervals and a temperature of the electrodes (12A, 12B);
f) calculating, by a processing unit, an electrical conductance value from the applied electrical voltage and the resulting measured voltage values, and
g) calculating, by the processing unit, a value relative to the hydric state of the plant by processing the calculated conductance value and the measured electrode temperature.

13. The method of claim 12, wherein the voltage measurement module (21) comprises a Wheatstone bridge (22) that allows the passage of electric current resulting from the electric voltage applied across it.

14. The method of claim 12 or 13, wherein:

- the first time interval and the second time interval have a fixed duration; or
- the application and inversion are performed until a stabilization of the electrical voltage in an area where the two electrodes (12A, 12B) are inserted.

**15.** The method of any one of claims 12 to 14, further comprising, simultaneously to step e), measurement of an ambient temperature near the plant and/or a measurement of an ambient humidity, light radiation, geolocation and/or a time stamp.

## Patentansprüche

**1.** System zur Bewertung des Wasserzustands einer Pflanze, umfassend:

- mindestens zwei Elektroden (12A, 12B), die in einem bestimmten Abstand voneinander getrennt und für das Einsetzen in eine gegebene Tiefe in den Stamm einer Pflanze angepasst sind, um einen galvanischen Kontakt mit der Pflanze aufzubauen;
- mindestens einen Temperatursensor der Elektroden (13);
- eine elektronische Einheit (10), die betriebsmäßig mit den zwei Elektroden (12A, 12B) und dem Temperatursensor der Elektroden (13) verbunden ist; und
- eine Verarbeitungseinheit, die einen Speicher und mindestens einen Prozessor beinhaltet, wobei das System **dadurch gekennzeichnet ist, dass**:
- die elektronische Einheit (10) ein Spannungsmessmodul (21) beinhaltet, das zu Folgendem konfiguriert ist:

- Anlegen einer elektrischen Spannung mit einer gegebenen Polarität an die mindestens zwei Elektroden (12A, 12B) während eines ersten Zeitintervalls;
- sequenzielles Invertieren der gegebenen Polarität während eines zweiten Zeitintervalls; und
- Messen von Spannungswerten, die aus dem Anlegen einer elektrischen Spannung in beiden Polaritäten resultieren, durchgeführt während des ersten und zweiten Zeitintervalls;

wobei der Temperatursensor der Elektroden (13) dazu konfiguriert ist, gleichzeitig mit der Messung eine Temperatur der Elektroden (12A, 12B) zu messen; und wobei der mindestens ein Prozessor zu Folgendem konfiguriert ist:
Berechnen eines elektrischen Leitfähigkeitswerts aus der angelegten elektrischen Spannung und den resultierenden gemessenen Spannungswerten und Ableiten eines Werts in Bezug auf den Wasserzustand der Pflanze durch Verarbeiten des berechneten Leitfähigkeitswerts und der gemessenen Elektrodentemperatur.

**2.** System nach Anspruch 1, wobei das Spannungsmessmodul (21) eine Wheatstone-Brücke (22) umfasst, die dazu angepasst ist, den Durchgang von elektrischem Strom zu ermöglichen, der aus der an sie angelegten elektrischen Spannung resultiert.

**3.** System nach Anspruch 1 oder 2, ferner umfassend einen Analog-Digital-Wandler (15), der dazu angepasst ist, vor der Verarbeitung die resultierenden Spannungswerte und die gemessene Elektrodentemperatur in äquivalente Digitalsignale umzuwandeln.

**4.** System nach einem der vorhergehenden Ansprüche, wobei die elektronische Einheit (10) ferner eine elektrische Leistungsversorgung (18) beinhaltet, einschließlich einer oder mehrerer Batterien, einer oder mehrerer photovoltaischer Platten oder eines oder mehrerer thermoelektrischer Generatoren.

**5.** System nach einem der vorhergehenden Ansprüche, ferner umfassend einen Umgebungstemperatursensor (14).

**6.** System nach einem der vorhergehenden Ansprüche, ferner umfassend eines oder mehrere der folgenden Elemente: einen Umgebungsfeuchtigkeitssensor, einen Lichtstrahlungssensor, ein Geopositionierungssystem, ein Überprüfungssystem für interne Betriebsfehler und/oder einen Echtzeit-Taktgeber.

**7.** System nach einem der vorhergehenden Ansprüche, wobei die Verarbeitungseinheit von der elektronischen Einheit (10) entfernt ist, wobei die Verarbeitungseinheit und die elektronische Einheit (10) jeweils ein Kommunikationsmodul beinhalten.

**8.** System nach einem der vorhergehenden Ansprüche 1 bis 6, wobei die Verarbeitungseinheit in der elektronischen Einheit (10) beinhaltet ist, wobei die elektronische Einheit (10) ferner ein Kommunikationsmodul beinhaltet.

**9.** System nach einem der vorhergehenden Ansprüche 1 bis 6, wobei die Verarbeitungseinheit von der elektronischen Einheit (10) entfernt ist und die elektronische Einheit (10) eine lokale Verarbeitungseinheit (17) beinhaltet, wobei beide Verarbeitungseinheiten mittels einer Kommunikationseinheit (19) der elektronischen Einheit (10) verbunden sind.

**10.** System nach einem der vorhergehenden Ansprüche, ferner umfassend ein Befestigungssystem (16) der elektronischen Einheit (10) am Stamm der Pflanze.

**11.** System nach einem der vorhergehenden Ansprüche, wobei die elektronische Einheit (10) eine Visualisierungsschnittstelle beinhaltet.

**12.** Verfahren zur Bewertung des Wasserzustands einer Pflanze, umfassend:

a) Einführen von mindestens zwei Elektroden (12A, 12B) in eine gegebene Tiefe in den Stamm einer Pflanze, wobei die zwei Elektroden (12A, 12B) einen galvanischen Kontakt mit der Pflanze aufrechterhalten und in einem bestimmten Abstand voneinander getrennt sind;

b) Einführen mindestens eines Temperatursensors der Elektroden (13);

c) Anlegen einer elektrischen Spannung mit einer gegebenen Polarität an die mindestens zwei Elektroden (12A, 12B) durch eine elektronische Einheit (10) während eines ersten Zeitintervalls;

d) sequenzielles Invertieren der gegebenen Polarität durch die elektronische Einheit (10) während eines zweiten Zeitintervalls;

e) Messen, durch die elektronische Einheit (10) unter Verwendung eines Spannungsmessmoduls (21) und des Temperatursensors der Elektroden (13), gleichzeitig von Spannungswerten, die aus dem Anlegen der elektrischen Spannung in beiden Polaritäten resultieren, durchgeführt während des ersten und zweiten Zeitintervalls, und einer Temperatur der Elektroden (12A, 12B);

f) Berechnen eines elektrischen Leitfähigkeitswerts aus der angelegten elektrischen Spannung und den resultierenden gemessenen Spannungswerten durch eine Verarbeitungseinheit und

g) Berechnen eines Werts in Bezug auf den Wasserzustand der Pflanze durch die Verarbeitungseinheit durch Verarbeiten des berechneten Leitfähigkeitswerts und der gemessenen Elektrodentemperatur.

**13.** Verfahren nach Anspruch 12, wobei das Spannungsmessmodul (21) eine Wheatstone-Brücke (22) umfasst, die den Durchgang von elektrischem Strom ermöglicht, der aus der an sie angelegten elektrischen Spannung resultiert.

**14.** Verfahren nach Anspruch 12 oder 13, wobei:

- das erste Zeitintervall und das zweite Zeitintervall eine feststehende Dauer aufweisen; oder
- das Anlegen und das Invertieren durchgeführt werden, bis sich die elektrische Spannung in einem Bereich, in dem die beiden Elektroden (12A, 12B) eingeführt sind, stabilisiert hat.

**15.** Verfahren nach einem der Ansprüche 12 bis 14, ferner umfassend, gleichzeitig mit Schritt e), die Messung einer Umgebungstemperatur nahe der Pflanze und/oder eine Messung einer Umgebungsfeuchtigkeit, Lichtstrahlung, Geolokalisierung und/oder eines Zeitstempels.

**Revendications**

**1.** Système pour l'évaluation de l'état hydrique d'une plante, comprenant :

- au moins deux électrodes (12A, 12B) séparés à une certaine distance l'un de l'autre et adaptés pour être insérés, jusqu'à une certaine profondeur, dans le tronc d'une plante pour établir un contact galvanique avec la plante ;
- au moins un capteur de température des électrodes (13) ;
- une unité électronique (10), opérativement reliée aux deux électrodes (12A, 12B) et au capteur de température des électrodes (13) ; et
- une unité de traitement comportant une mémoire et au moins un processeur, le système étant **caractérisé en ce que** :
- l'unité électronique (10) comporte un module de mesure de tension (21) configuré pour :

- appliquer, pendant un premier intervalle de temps, une tension électrique, avec une polarité donnée, sur les au moins deux électrodes (12A, 12B) ;
- inverser, séquentiellement, pendant un deuxième intervalle de temps, ladite polarité donnée ; et
- mesurer des valeurs de tension résultantes de ladite application de tension électrique dans les deux polarités réalisée pendant lesdits premier et deuxième intervalles de temps ;

dans lequel le capteur de température des électrodes (13) est configuré pour mesurer, simultanément avec ladite mesure, une température des électrodes (12A, 12B) ; et

dans lequel l'au moins un processeur est configuré pour :

calculer une valeur de conductance électrique à partir de la tension électrique appliquée et les valeurs de tension mesurés résultantes, et

en déduire une valeur relative à l'état hydrique de la plante par le biais du traitement de la valeur de conductance calculée et la température d'électrode mesurée.

**2.** Système selon la revendication 1, dans lequel le module de mesure de tension (21) comprend un pont de Wheatstone (22) adapté pour permettre le passage de courant électrique résultant de la tension électrique appliqué à travers celui-ci.

3. Système selon la revendication 1 ou 2, comprenant en outre un convertisseur analogique-numérique (15) adapté pour convertir, avant le traitement, lesdites valeurs de tension résultantes et la température d'électrode mesurée en signaux numériques équivalents.

4. Système selon l'une quelconque des revendications précédentes, dans lequel l'unité électronique (10) comprend en outre une alimentation de puissance électrique (18) comportant une ou plusieurs batteries, un ou plusieurs panneaux photovoltaïques, ou un ou plusieurs générateurs thermoélectriques.

5. Système selon l'une quelconque des revendications précédentes, comprenant en outre un capteur de température ambiante (14).

6. Système selon l'une quelconque des revendications précédentes, comprenant en outre un ou plusieurs des éléments suivants : un capteur d'humidité ambiante, un capteur de rayonnement lumineux, un système de géolocalisation, un système de vérification d'erreurs opérationnel interne et/ou une horloge en temps réel.

7. Système selon l'une quelconque des revendications précédentes, dans lequel l'unité de traitement est distante de l'unité électronique (10), dans lequel l'unité de traitement et l'unité électronique (10) comportent, chacune, un module de communications.

8. Système selon l'une quelconque des revendications précédentes 1 à 6, dans lequel l'unité de traitement est incluse dans l'unité électronique (10), dans lequel l'unité électronique (10) comporte en outre un module de communications.

9. Système selon l'une quelconque des revendications précédentes 1 à 6, dans lequel l'unité de traitement est distante de l'unité électronique (10) et l'unité électronique (10) comporte une unité de traitement locale (17), les deux unités de traitement étant reliées par le biais d'une unité de communications (19) de l'unité électronique (10).

10. Système selon l'une quelconque des revendications précédentes, comprenant en outre un système de fixation (16) de l'unité électronique (10) au tronc de la plante.

11. Système selon l'une quelconque des revendications précédentes, dans lequel l'unité électronique (10) comporte une interface de visualisation.

12. Procédé pour l'évaluation de l'état hydrique d'une plante, comprenant :

a) insérer, à une profondeur donnée, au moins deux électrodes (12A, 12B) dans le tronc d'une plante, les deux électrodes (12A, 12B) maintenant un contact galvanique avec la plante et étant séparés une certaine distance l'un de l'autre ;
b) insérer au moins un capteur de température des électrodes (13) ;
c) appliquer, par une unité électronique (10), pendant un premier intervalle de temps, une tension électrique, avec une polarité donnée, sur les au moins deux électrodes (12A, 12B) ;
d) inverser, par l'unité électronique (10), séquentiellement, pendant un deuxième intervalle de temps, ladite polarité donnée ;
e) mesurer, par l'unité électronique (10) en utilisant un module de mesure de tension (21) et le capteur de température des électrodes (13), simultanément, des valeurs de tension résultantes de ladite application de tension électrique dans les deux polarités réalisée pendant lesdits premier et deuxième intervalles de temps et la température des électrodes (12A, 12B) ;
f) calculer, par une unité de traitement, une valeur de conductance électrique à partir de la tension électrique appliquée et les valeurs de tension mesurées résultantes, et
g) calculer, par l'unité de traitement, une valeur relative à l'état hydrique de la plante par le biais du traitement de la valeur de conductance calculée et la température d'électrode mesurée.

13. Procédé selon la revendication 12, dans lequel le module de mesure de tension (21) comprend un pont de Wheatstone (22) qui permet le passage de courant électrique résultant de la tension électrique appliqué à travers celui-ci.

14. Procédé selon la revendication 12 ou 13, dans lequel :

- le premier intervalle de temps et le deuxième intervalle de temps ont une durée fixe ; ou
- l'application et inversion sont réalisées jusqu'à une stabilisation de la tension électrique dans une zone où les deux électrodes (12A, 12B) sont insérées.

15. Procédé selon l'une quelconque des revendications 12 à 14, comprenant en outre, simultanément à l'étape e), la mesure d'une température ambiante près de la plante et/ou la mesure d'une humidité ambiante, rayonnement lumineux, géolocalisation et/ou un horodatage.

**FIG. 1**

**101**

Insert two electrodes in the trunk of a plant

**102**

Insert temperature sensor

**103**

Apply a voltage with a given polarity and sequentially reverse that polarity and measure a voltage and a temperature of the electrodes

**104**

Obtain the value relative to the hydric state of the plant

## FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9857391 B2
- JP H06273434 B
- KR 101996404 B1
- CN 111189885 A
- CN 113418963 A
- WO 2022039007 A